# EUROPÄISCHE PATENTSCHRIFT

(11) **EP 0 600 310 B1**
(45) Veröffentlichungstag und Bekanntmachung des Hinweises auf die Patenterteilung: **01.05.1996**
(21) Anmeldenummer: 93118555.7
(22) Anmeldetag: 18.11.1993
(51) Int. Cl.: C07C 265/04, C07C 265/10, C07D 251/34, C07C 271/24

(54) **Polyisocyanatgemische, ein Verfahren zu ihrer Herstellung und ihre Verwendung**
Polyisocyanate mixtures, method for their preparation and their use
Mélanges des polyisocyanates, procédé pour leur préparation et leur utilisation

(30) Priorität: 01.12.1992 DE 4240330
(43) Veröffentlichungstag der Anmeldung: 08.06.1994
(73) Patentinhaber: BAYER AG, D-51368 Leverkusen (DE)
(72) Erfinder: Laas, Hans Josef, Dr., D-50793 Köln (DE); Halpaap, Reinhard, Dr., D-51519 Odenthal (DE); Wamprecht, Christian, Dr., D-41472 Neuss (DE); Meier-Westhues, Hans-Ulrich, Dr., D-51379 Leverkusen (DE); Schultz, Wolfgang, D-47829 Krefeld (DE); Kahl, Lothar, Dr., D-51465 Bergisch Gladbach (DE)

(56) Entgegenhaltungen:
- EP-A- 0 045 994
- EP-A- 0 254 152
- EP-A- 0 465 302
- DE-A- 1 815 311
- US-A- 4 159 376

## Beschreibung

Die Erfindung betrifft neue, unblockierte, als Vernetzer für Pulverlacke geeignete Polyisocyanatgemische, ein Verfahren zu ihrer Herstellung und ihre Verwendung zur Herstellung von Polyurethankunststoffen, insbesondere als Härter für Polyurethan-Pulverlacke.

Bei Raumtemperatur feste Kombinationen aus organischen Polyhydroxylverbindungen und blockierten Polyisocyanaten stellen wertvolle Bindemittel für Hitzevernetzbare Pulverlacke dar (vgl. z.B. DE-OS 21 05 777, DE-OS 25 42 191, DE-OS 31 43 060, DE-OS 27 35 497, DE-OS 28 42 641, EP-A-0 286 799, EP-A-0 218 040, EP-A-0 460 963, DE-OS 28 01 126, EP-A-0 403 779, WO 91/15532, US-PS 3 857 818, US-PS 4 375 539, EP-A-0 409 745, DE-OS 28 12 252, DE-OS 29 46 085 oder DE-OS 34 34 881).

Den Systemen dieser Vorveröffentlichungen gemeinsam ist der Nachteil, daß bei der thermischen Vernetzung die als Blockierungsmittel eingesetzten Verbindungen abgespalten werden und in die Umwelt entweichen. Bei ihrer Verarbeitung müssen daher aus Gründen der Ökologie und Arbeitshygiene besondere Vorkehrungen zur Reinigung der Abluft und/oder Wiedergewinnung des Blockierungsmittels getroffen werden.

Ein Versuch, diesen prinzipiellen Nachteil zu beseitigen, ist in der Verwendung von Blockierungsmittelfreien, linearen Uretdion- und Urethangruppen-haltigen IPDI-Pulverlackhärtern zu sehen, die endständig Urethan-, Harnstoff- oder freie Isocyanatgruppen aufweisen (EP-A-0 045 994, EP-A-0 045 996 und EP-A-0 045 998) und bei denen die Vernetzung unter thermischer Rückspaltung der Uretdiongruppen erfolgt. Als nachteilig erweist sich bei diesen Vernetzern jedoch ihre strikte Linearität, die für das Syntheseverfahren erforderlich ist, und die eine Verzweigung des Lacks zur Einstellung einer guten Lösemittelfestigkeit, Kratzfestigkeit und hohen Härte durch Variation des Härters nicht erlaubt.

Die EP-A-0 193 828, EP-A-0 224 165 und EP-A-0 254 152 beschreiben spezielle bei Raumtemperatur feste, Isocyanurat- und/oder Urethangruppen-haltige Polyisocyanate mit freien, an tertiäre (cyclo)aliphatische Kohlenstoffatome gebundenen Isocyanatgruppen als Vernetzerkomponenten für PUR-Pulverlacke. Aufgrund der Reaktionsträgheit der tertiär gebundenen NCO-Gruppen lassen sich diese Polyisocyanate in unblockierter Form mit OH-funktionellen Pulverlackbindemitteln bei Temperaturen oberhalb ihres Schmelzpunktes vermischen, ohne daß eine unerwünschte Vorreaktion eintritt. Auf diese Weise wird die Herstellung lagerstabiler, blockierungsmittelfreier PUR-Pulverlacke ermöglicht, die bei 150 bis 220°C, vorzugsweise 170 bis 190°C zu hochvernetzten, glänzenden Lackfilmen mit guter Härte, Elastizität und Lösemittelbeständigkeit vernetzen.

Im Bestreben die Lösemittelemissionen aus Beschichtungsmitteln weiter zu reduzieren, wird heute auch für eine Reihe von Anwendungen, die bislang noch konventionellen lösemittelhaltigen oder wäßrigen Lacksystemen vorbehalten sind, beispielsweise für die Automobilerstlackierung, der Einsatz von Pulverlacken diskutiert. Dabei besteht insbesondere ein Bedarf an Pulverlacksystemen, die unterhalb 150°C insbesondere im Bereich von ca. 140°C ohne Abspaltung von Blockierungsmitteln aushärtbar sind, eine Anforderung, der die obengenannten blockierungsmittelfreien Polyisocyanatvernetzer mit freien tertiär gebundenen Isocyanatgruppen nicht genügen.

Es war daher die der vorliegenden Erfindung zugrundeliegende Aufgabe, neue, als Härter für Pulverlacke geeignete, unblockierte Polyisocyanate zur Verfügung zu stellen, die eine Vernetzung der Lacke bei Einbrenntemperaturen von unter 150°C ermöglichen und dabei lösemittel- und chemikalienbeständige Lackfilme mit guten optischen und mechanischen Eigenschaften ergeben.

Diese Aufgabe konnte mit der Bereitstellung der nachfolgend näher beschriebenen erfindungsgemäßen Polyisocyanatgemische bzw. des Verfahrens zu ihrer Herstellung gelöst werden. Die nachfolgend näher beschriebenen erfindungsgemäßen Polyisocyanatgemische basieren auf der überraschenden Beobachtung, daß sich durch den Einbau von Ester- und/oder Carbonatgruppen in Polyisocyanate mit an tertiäre Kohlenstoffatome gebundenen Isocyanatgruppen die Reaktivität dieser Isocyanatgruppen so weit steigern läßt, daß die Herstellung blockierungsmittelfreier Pulverlacke ermöglicht wird, die bei Einbrenntemperaturen von deutlich unter 150°C, z.B. im Bereich von 120 bis 140°C, vernetzen.

Gegenstand der Erfindung sind somit Polyisocyanatgemische die unterhalb von 40°C in fester und oberhalb von 125°C in flüssiger Form vorliegen und
a) eine mittlere Isocyanatfunktionalität von mindestens 2,1,
b) einen Gehalt an freien, an tertiäre (cyclo)aliphatische Kohlenstoffatome gebundenen Isocyanatgruppen (berechnet als NCO; Molekulargewicht = 42) von 5 bis 22 Gew.-%,
c) einen Gehalt an Urethangruppen (berechnet als -NH-CO-O-; Molekulargewicht = 59) von 2 bis 30 Gew.-% und
d) einen Gehalt an Isocyanuratgruppen (berechnet als C₃N₃O₃; Molekulargewicht = 126) von 0 bis 30 Gew.-%
aufweisen, dadurch gekennzeichnet, daß die Polyisocyanatgemische zusätzlich
e) einen Gehalt an Carbonsäureestergruppen (berechnet als -CO-O-, Molekulargewicht = 44) von 0 bis 25 Gew.-% und/oder
f) einen Gehalt an Carbonatgruppen (berechnet als -O-CO-O-; Molekulargewicht = 60) von 0 bis 34 Gew.-% aufweisen,
mit der Maßgabe, daß der Gesamtgehalt an Carbonsäureester- und Carbonatgruppen mindestens 2 Gew.-% beträgt.

Gegenstand der Erfindung ist auch ein Verfahren zur Herstellung dieser Polyisocyanatgemische, das dadurch gekennzeichnet ist, daß man
A) Diisocyanate, die mindestens eine an ein tertiäres (cyclo)aliphatisches Kohlenstoffatom gebundene Isocyanatgruppe aufweisen, gegebenenfalls unter Mitverwendung von
B) weiteren Diisocyanaten mit ausschließlich an primären und/oder sekundären (cyclo)aliphatischen Kohlenstoffatomen gebundenen Isocyanatgruppen mit
C) Estergruppen und/oder Carbonatgruppen aufweisenden Polyhydroxylverbindungen eines mittleren Molekulargewichtes von 134 bis 1200, gegebenenfalls unter gleichzeitiger Mitverwendung von
D) Estergruppen- und Carbonatgruppen-freien Polyhydroxylverbindungen des Molekulargewichtsbereiches 62 bis 400,
unter Einhaltung eines NCO/OH-Äquivalentverhältnisses von 1,2:1 bis 40:1 bis zum Erreichen des theoretisch berechneten NCO-Gehaltes umsetzt, gegebenenfalls im Anschluß daran die Umsetzungsprodukte durch katalytische Trimerisierung eines Teils der verbliebenen Isocyanatgruppen weiter modifiziert und/oder gegebenenfalls überschüssiges, nicht umgesetztes Diisocyanat durch Dünnschichtdestillation abtrennt, wobei im übrigen Art und Mengenverhältnisse der genannten Ausgangsstoffe so gewählt werden, daß die resultierenden Verfahrensprodukte den oben unter a) bis f) genannten Bedingungen entsprechen.

Gegenstand der Erfindung ist auch die Verwendung dieser Polyisocyanatgemische bei der Herstellung von Polyurethankunststoffen, insbesondere als Vernetzerkomponente in hitzehärtbaren Zweikomponenten-Polyurethan-Pulverlacken bei der Beschichtung beliebiger hitzeresistenter Substrate nach den Methoden der Pulverlacktechnologie.

Ausgangsverbindungen A) für das erfindungsgemäße Verfahren sind beliebige, mindestens eine an ein tertiäres (cyclo)aliphatisches Kohlenstoffatom gebundene Isocyanatgruppe aufweisende Diisocyanate. Geeignete Ausgangsdiisocyanate sind beispielsweise:
A1) aliphatische Diisocyanate mit einem NCO-Gehalt von 24 bis 50 Gew.-%, vorzugsweise 31 bis 50 Gew.-%, die neben einer sterisch ungehinderten, an ein primäres aliphatisches Kohlenstoffatom gebundenen Isocyanatgruppe eine sterisch gehinderte Isocyanatgruppe, die an ein tertiäres aliphatisches Kohlenstoffatom gebunden ist, aufweisen, oder
A2) aliphatisch-cycloaliphatische Diisocyanate mit einem NCO-Gehalt von 20 bis 50 Gew.-%, vorzugsweise 30 bis 48 Gew.-%, die neben einer sterisch ungehinderten, an ein primäres aliphatisches Kohlenstoffatom gebundenen Isocyanatgruppe eine sterisch gehinderte Isocyanatgruppe, die an ein tertiäres Kohlenstoffatom, das Teil eines cycloaliphatischen Ringsystems ist, gebunden ist, aufweisen, oder
A3) zwei Isocyanatoalkyl-Substituenten aufweisende aromatische Verbindungen mit einem NCO-Gehalt von 20 bis 35 Gew.-%, vorzugsweise 25 bis 35 Gew.-%, bei denen beide Isocyanatgruppen an tertiäre aliphatische Kohlenstoffatome gebunden sind.

Geeignete Diisocyanate A1) sind beispielsweise solche der allgemeinen Formel 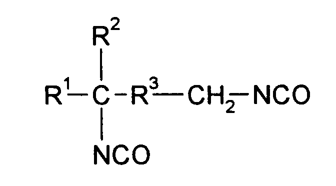
in welcher
- R¹ und R²: für gleiche oder verschiedene Reste stehen und Alkylgruppen mit 1 bis 4 Kohlenstoffatomen bedeuten und
- R³: für einen zweiwertigen, gegebenenfalls verzweigten, gesättigten aliphatischen Kohlenwasserstoffrest mit 2 bis 9 Kohlenstoffatomen steht.

Derartige Diisocyanate bzw. ihre Herstellung sind beispielsweise in den DE-OSen 36 08 354 und 36 20 821 beschrieben. Bevorzugte Diisocyanate A1) sind solche, in welchen R¹ und R² jeweils für Methylreste stehen. Typische Vertreter sind beispielsweise 1,4-Diisocyanato-4-methylpentan, 1,5-Diisocyanato-5-methylhexan, 1,6-Diisocyanato-6-methylheptan, 1,5-Diisocyanato-2,2,5-trimethylhexan oder 1,7-Diisocyanato-3,7-dimethyloctan.

Geeignete Ausgangsdiisocyanate A2) sind solche der allgemeinen Formel 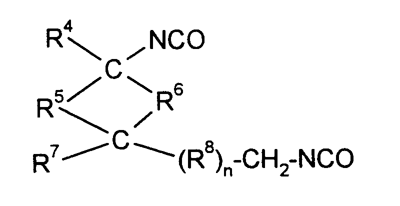
in welcher
- R⁴: für einen Alkylrest mit 1 bis 4 Kohlenstoffatomen, vorzugsweise einen Methylrest steht,
- R⁵ und R⁶: für gleiche oder verschiedene Reste stehen und jeweils einen zweiwertigen linearen oder verzweigten gesättigten Kohlenwasserstoffrest mit 1 bis 4, vorzugsweise 1 bis 3 Kohlenstoffatomen bedeuten, wobei die Summe der Kohlenstoffatome dieser Reste vorzugsweise 3 bis 6, insbesondere 4 oder 5 beträgt,
- R⁷: für Wasserstoff oder einen Alkylrest mit 1 bis 4 Kohlenstoffatomen, vorzugsweise für Wasserstoff oder einen Methylrest steht,
- R⁸: für einen zweiwertigen, linearen oder verzweigten, gesättigten aliphatischen Kohlenwasserstoffrest mit 1 bis 4, insbesondere 1 bis 3 Kohlenstoffatomen steht und
- n: für 0 oder 1 steht.

Die Herstellung derartiger aliphatisch-cycloaliphatischer Diisocyanate ist beispielsweise in EP-A-0 153 561 beschrieben.

Bevorzugte Ausgangsdiisocyanate A2) sind beispielsweise 1-Isocyanato-1-methyl-4(3)-isocyanatomethylcyclohexan, welches im allgemeinen als Gemisch der 4- und 3-Isocyanatomethyl-Isomeren vorliegt, 1-Isocyanato-1-methyl-4-(4-isocyanatobut-2-yl)-cyclohexan, 1-Isocyanato-1,2,2-trimethyl-3-(2-isocyanatoethyl)-cyclopentan oder 1-Isocyanato-1,4(3)-dimethyl-4(3)-isocyanatomethylcyclohexan, welches im allgemeinen in Form eines 4-Methyl-4-isocyanatomethyl- und 3-Methyl-3-isocyanatomethyl-Isomerengemischs vorliegt. Geeignet sind jedoch auch z.B. 1-Isocyanato-1-butyl-3-(4-isocyanatobut-1-yl)-cyclopentan, 1-Isocyanato-1-ethyl-4-butyl-4-(4-isocyanatobut-1-yl)-cyclohexan oder 1-Isocyanato-1,2-dimethyl-3-ethyl-3-isocyanatmethyl-cyclopentan.

Geeignete Ausgangsdiisocyanate A3) sind beispielsweise solche der allgemeinen Formel 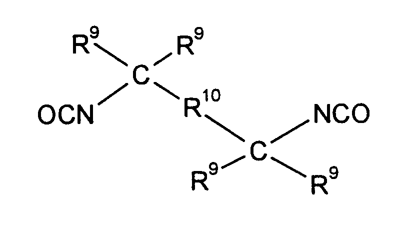
in welcher
- R⁹: für einen Alkylrest mit 1 bis 4 Kohlenstoffatomen, vorzugsweise einen Methylrest, steht und
- R¹⁰: für einen zweiwertigen aromatischen Kohlenwasserstoffrest steht, ausgewählt aus der Reihe der Phenylen-, Biphenylen- oder Naphthylen-Reste, die gegebenenfalls Halogen-, Methyl- oder Methoxysubstituiert sein können.

Derartige Diisocyanate sind ebenfalls bekannt. Ihre Herstellung wird beispielsweise in der EP-A-0 101 832 beschrieben.

Bevorzugte Diisocyanate A3) sind 1,3- oder 1,4-Bis-(2-isocyanato-prop-2-yl)-benzol.

Als Ausgangskomponente A) für das erfindungsgemäße Verfahren können selbstverständlich auch beliebige Gemische der beispielhaft untr A1), A2) und A3) aufgeführten Diisocyanate zum Einsatz kommen.

Bevorzugte Ausgangsdiisocyanate A) sind die vorstehend beschriebenen aliphatisch-cycloaliphatischen Diisocyanate A2). Ganz besonders bevorzugt ist die Verwendung von 1-Isocyanato-1-methyl-4(3)-isocyanatomethylcyclohexan.

Gegebenenfalls können beim erfindungsgemäßen Verfahren weitere Diisocyanate B) mit ausschließlich an primären und/oder sekundären (cyclo)aliphatischen Kohlenstoffatomen gebundenen Isocyanatgruppen mitverwendet werden. Es handelt sich hierbei vorzugsweise um solche des Molekulargewichtsbereiches 140 bis 400, wie z.B. 1,4-Diisocyanatobutan, 1,6-Diisocyanatohexan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1-Isocyanato-3,3,5-trimethyl-5-isocyanatomethylcyclohexan (Isophorondiisocyanat) und 4,4'-Diisocynatodicyclohexylmethan oder beliebige Gemische solcher Diisocyanate. Besonders bevorzugtes Diisocyanat B) ist 1,6-Diisocyanatohexan.

Im Falle einer Mitverwendung können die Diisocyanate B) beim erfindungsgemäßen Verfahren in Mengen von bis zu 25 Mol-%, bezogen auf die Gesamtmenge der Ausgangskomponenten A) und B) zum Einsatz kommen.

Beim erfindungsgemäßen Verfahren kommen Estergruppen und/oder Carbonatgruppen-aufweisende Polyhydroxylverbindungen C) eines mittleren, aus Funktionalität und Hydroxylzahl berechenbaren Molekulargewichtes von 134 bis 2500, vorzugsweise 176 bis 800, und einer mittleren OH-Funktionalität von 2,0 bis 4,0, vorzugsweise 2,2 bis 3,8 zum Einsatz.

Hierbei handes es sich beispielsweise um die an sich bekannten Esteralkohole oder Esteralkohol-Gemische, wie sie sich z.B. durch Umsetzung von mehrwertigen Alkoholen mit unterschüssigen Mengen an mehrwertigen Carbonsäuren, entsprechenden Carbonsäureanhydriden, entsprechenden Polycarbonsäureestern von niederen Alkoholen oder Lactonen herstellen lassen.

Zur Herstellung dieser Esteralkohole geeignete mehrwertige Alkohole sind insbesondere solche des Molekulargewichtsbereichs 62 bis 400 wie z.B. 1,2-Ethandiol, 1,2- und 1,3-Propandiol, die isomeren Butandiole, Pentandiole, Hexandiole, Heptandiole und Octandiole, 1,2- und 1,4-Cyclohexandiol, 1,4-Cyclohexandimethanol, 4,4'-(1-Methylethyliden)-biscyclohexanol, 1,2,3-Propantriol, 1,1,1-Trimethylolethan, 1,2,6-Hexandiol, 1,1,1-Trimethylolpropan, 2,2-Bis(hydroxymethyl)-1,3-propandiol oder 1,3,5-Tris(2-hydroxyethyl)-isocyanurat.

Die zur Herstellung der Esteralkohole verwendeten Säuren oder Säurederivate können aliphatischer, cycloaliphatischer, aromatischer und/oder heteroaromatischer Natur sein und gegebenenfalls, z.B. durch Halogenatome, substituiert und/oder ungesättigt sein. Beispiele geeigneter Säuren sind beispielsweise mehrwertige Carbonsäuren des Molekulargewichtsbereichs 118 bis 300 oder deren Derivate wie beispielsweise Bernsteinsäure, Adipinsäure, Sebacinsäure, Phthalsäure, Isophthalsäure, Trimellitsäure, Phthalsäureanhydrid, Tetrahydrophthalsäure, Maleinsäure, Maleinsäureanhydrid, dimere und trimere Fettsäuren, Terephthalsäuredimethylester und Terephthalsäure-bis-glykolester.

Zur Herstellung der Esteralkohole können auch beliebige Gemische der beispielhaft genannten Ausgangsverbindungen eingesetzt werden. Weiterhin ist es möglich beim erfindungsgemäßen Verfahren Gemische verschiedener Esteralkohole der genannten Alt zu verwenden.

Bevorzugt kommen beim erfindungsgemäßen Verfahren als Ausgangskomponente C) jedoch Esterpolyole zum Einsatz, wie sie sich in an sich bekannter Weise aus Lactonen und einfachen mehrwertigen Alkoholen als Startermolekülen unter Ringöffnung herstellen lassen.

Geeignete Lactone zur Herstellung dieser Esterpolyole sind beispielsweise β-Propiolacton, γ-Butyrolacton, δ-Valerolacton, ε-Caprolacton, 3,5,5- und 3,3,5-Trimethylcaprolacton oder beliebige Gemische solcher Lactone. Als Startermoleküle dienen beispielsweise die oben beispielhaft genannten mehrwertigen Alkohole des Molekulargewichtsbereichs 62 bis 400 oder beliebige Gemische dieser Alkohole.

Besonders bevorzugte Estergruppen aufweisende Polyhydroxylvrbindungen C) für das erfindungsgemäße Verfahren sind Esterpolyole des obengenannten Molekulargewichtsbereiches auf Basis von ε-Caprolacton, insbesondere solche, bei deren Herstellung 1,1,1-Trimethylolpropan als Startermolekül verwendet wurde.

Ausgangsverbindungen C) für das erfindungsgemäße Verfahren stellen auch Carbonatgruppen aufweisende Polyhydroxylverbindungen dar. Hierbei handelt es sich um Carbonatalkohole der an sich bekannten Art, wie sie beispielsweise durch Umsetzung der oben beispielhaft genannten mehrwertigen Alkohole des Molekulargewichtsbereiches 62 bis 400 mit Diarylcarbonaten, wie z.B. Diphenylcarbonat, Phosgen oder bevorzugt cyclischen Carbonaten, wie z.B. Trimethylencarbonat oder 2,2-Dimethyl-trimethylencarbonat (Neopentylglykolcarbonat, NPC) oder Gemischen solcher cyclischen Carbonate, erhalten werden können. Besonders bevorzugte Carbonatalkohole sind solche, die aus den genannten mehrwertigen Alkoholen als Startermoleküle und NPC unter Ringoffnung hergestellt werden können.

Als Ausgangsverbindungen C) für das erfindungsgemäße Verfahren eignen sich weiterhin auch Estergruppen- und Carbonatgruppen-aufweisende Polyhydroxylverbindungen. Solche Estercarbonatalkohole sind beispielsweise gemäß der Lehre DE-AS 17 70 245 durch Umsetzung der oben beispielhaft genannten mehrwertigen Alkohole des Molekulargewichtsbereiches 62 bis 400 mit Lactonen der oben beispielhaft genannten Art, insbesondere ε-Caprolacton und anschließende Reaktion der dabei entstehenden Esteralkohole mit Diphenylcarbonat herstellbar. Bevorzugt kommen jedoch Estercarbonatalkohole zum Einsatz, wie sie sich durch Umsetzung der genannten mehrwertigen Alkohole mit Mischungen von Lactonen und cyclischen Carbonaten unter Ringöffnung erhalten lassen.

Die Herstellung der vorstehend beschriebenen, bevorzugt beim erfindungsgemäßen Verfahren eingesetzten Esteralkohole, Carbonatalkohole und Estercarbonatalkohole durch ringöffnende Polymerisation, erfolgt im allgemeinen in Gegenwart von Katalysatoren wie beispielsweise Lewis- oder Brönstedt-Säuren, organischen Zinn- oder Titanverbindungen bei Temperaturen von 20 bis 200°C, vorzugsweise 50 bis 160°C.

Beliebige Gemische der beispielhaft genannten Esteralkohole, Carbonatalkohole und Estercarbonatalkohole können gegebenenfalls beim erfindungsgemäßen Verfahren als Ausgangskomponente C) zum Einsatz gelangen.

Gegebenenfalls können beim erfindungsgemäßen Verfahren auch Estergruppen- und Carbonatgruppen-freie Polyhydroxylverbindungen D) des Molekulargewichtsbereiches 62 bis 400 mitverwendet werden. Hierbei handelt es sich beispielsweise um die oben bei der Herstellung der Esteralkohole beschriebenen einfachen mehrwertigen Alkohole oder beliebige Gemische dieser Alkohole. Estergruppen- und Carbonatgruppen-freie Alkohole D) werden, falls überhaupt, in Mengen von bis zu 80 Gew.-% bezogen auf die Gesamtmenge der Ausgangskomponenten C) und D) mitverwendet. Für das erfindungsgemäße Verfahren geeignete Gemische aus Ausgangskomponenten C) und D) resultieren beispielsweise auch dann, wenn die als Ausgangsmaterial eingesetzten mehrwertigen Alkohole der beispielhaft genannten Art mit unterschüssigen Mengen an Säuren bzw. Säurederivaten, Diarylcarbonaten oder cyclischen Carbonaten der beispielhaft genannten Alt oder Phosgen nur teilweise in Estergruppen und/oder Carbonatgruppen-haltige Polyhydroxylverbindungen überführt werden.

Zur Durchführung des erfindungsgemäßen Verfahrens werden die Ausgangsdiisocyanate A), gegebenenfalls unter Mitverwendung weiterer Diisocyanate B), mit Estergruppen und/oder Carbonatgruppen-aufweisenden Polyhydroxylverbindungen C) und gegebenenfalls Estergruppen- und Carbonatgruppen-freien Polyhydroxylverbindungen D) in dem genannten Äquivalentverhältnis von NCO/OH = 1,2:1 bis 40:1, vorzugsweise von 1,4:1 bis 20:1 bis zum theoretisch berechneten NCO-Gehalt umgesetzt, wobei durch Auswahl der Polyhydroxylkomponente C) und gegebenenfalls D) dafür Sorge getragen wird, daß die resultierenden, Urethangruppen aufweisenden Polyisocyanate eine (mittlere) NCO-Funktionalität von mindestens 2,1, vorzugsweise von 2,1 bis 5,0 und insbesondere von 2,2 bis 4,8 aufweisen, es sei denn, im Anschluß an die genannte Umsetzung erfolgt, wie weiter unten beschrieben, eine Trimerisierungsreaktion, die zu einer Erhöhung der NCO-Funktionalität führt. In einem solchen Falle können als Polyhydroxylkomponenten C) und D) auch ausschließlich zweiwertige Verbindungen eingesetzt werden, während im erstgenannten Falle Gemische aus zwei- und höherwertigen Polyolkomponenten bzw. ausschließlich höher als zweiwertige Polyolkomponenten verwendet werden.

Die zur Urethanbildung erforderliche Reaktionstemperatur liegt beim erfindungsgemäßen Verfahren bei 20 bis 200°C, bevorzugt bei 40 bis 160°C, besonders bevorzugt bei 40 bis 120°C. Die Umsetzung wird bevorzugt ohne Lösungsmittel durchgeführt. Zur Beschleunigung der Urethanisierungsreaktion können die üblichen aus der Polyurethanchemie bekannten Katalysatoren eingesetzt werden, beispielsweise tert. Amine wie Triethylamin, Pyridin, Methylpyridin, Benzyldimethylamin, N,N-Endoethylenpiperazin, N-Methylpiperidin, Pentamethyldiethylentriamin, N,N-Dimethylaminocyclohexan, N,N'-Dimethylpiperazin oder Metallsalze wie Eisen(III)-chlorid, Zinkchlorid, Zink-2-ethylcaproat, Zinn-(II)-ethylcaproat, Dibutylzinn(IV)-dilaurat und Molybdänglykolat.

Diese Katalysatoren kommen gegebenenfalls in Mengen von 0,001 bis 2,0 Gew.-%, bevorzugt 0,01 bis 0,2 Gew.-%, bezogen auf die Gesamtmenge der verwendeten Ausgangsverbindungen zum Einsatz.

Bei der Verwendung der Diisocyanate A3) mit zwei sterisch gehinderten tertiären Isocyanatgruppen schließt sich in der Regel eine Dünnschichtdestillation an, um durch Abdestillieren des überschüssigen monomeren Diisocyanats monomerenarme erfindungsgemäße Polyisocyanatgemische mit Monomerengehalten <0,5 Gew.-% zu erhalten.

Beim Einsatz der Diisocyanate A1) und A2) mit einer sterisch gehinderten tertiären und einer sterisch ungehinderten primären Isocyanatgruppe als Ausgangsdiisocyanate für das erfindungsgemäße Verfahren kann auf eine Dünnschichtdestillation verzichtet werden. Bei Einhaltung eines Äquivalentverhältnisses von NCO/OH = 1,2:1 bis 2:1, bevorzugt 1,4:1 bis 2:1, werden aufgrund der um Größenordnungen geringeren Reaktivität der tertiären Isocyanatgruppen im Vergleich zu den primären Isocyanatgruppen der Ausgangsdiisocyanate A1) und A2) bzw. zu den primären und/oder sekundären Isocyanatgruppen der Diisocyanate B) ohne eine weitere Operation direkt monomerenarme Polyisocyanatgemische mit einem Monomerengehalt <1 %, im allgemeinen <0,5 %, erhalten.

Werden die Diisocyanate A1) und/oder A2), gegebenenfalls unter Mitverwendung weiterer Diisocyanate B) in einem Äquivalentverhältnis von NCO/OH = 2:1 bis 40:1, vorzugsweise 2:1 bis 20:1 mit den Hydroxylkomponenten C) und gegebenenfalls D) umgesetzt, wobei die gegebenenfalls mitverwendeten Diisocyanate B) dem Reaktionsgemisch auch erst nach der Urethanbildung aus A1) und A2) sowie C) und gegebenenfalls D) einverleibt werden können, so kann auch in diesen Fällen eine Dünnschichtdestillation unterbleiben, wenn die nach der Urethanbildung verbliebenen sterisch ungehinderten an ein primäres oder sekundäres Kohlenstoffatom gebundenen Isocyanatgruppen durch Cyclotrimerisierung in Isocyanuratgruppen überführt werden. Die resultierenden Isocyanuratgruppen enthaltenden Polyisocyanatgemische werden monomerenarm erhalten, mit <1 Gew.%, bevorzugt <0,5 Gew.-% monomerem Diisocyanat.

Geeignete Trimerisierungskatalysatoren für das erfindungsgemäße Verfahren sind alle bislang zur Herstellung von Isocyanuratpolyisocyanaten eingesetzten Verbindungen wie beispielsweise Phosphine der in DE-OS 19 34 763 beschriebenen Art, Alkaliphenolate der in GB-PS 13 91 066 oder GB-PS 13 86 399 beschriebenen Art, Aziridinderivate in Kombination mit tertiären Aminen der in DE-OS 23 25 826 beschriebenen Art, quaternäre Ammoniumcarboxylate der in EP-OS 17 998 beschriebenen Art, quaternäre Ammoniumphenolate mit zwitterionischer Struktur der beispielsweise in US-PS 43 35 219 beschriebenen Art, Ammoniumphosphonate und -phosphate der in DE-OS 32 27 489 beschriebenen Art, Alkalicarboxylate der in DE-OS 32 19 608 beschriebenen Art, siliziumorganische Verbindungen der in den EP-A'en 57 653, 89 297 und 187 105 beschriebenen Art, Ammoniumfluoride der in EP-A 339 396 beschriebenen Art sowie basische Alkalimetallsalze in Verbindung mit Phasentransfer-Katalysatoren, wie sie von R. Richter, P. Müller und K. Wagner, "Die Angewandte Makromolekulare Chemie", 113, 1-9 (1983) näher beschrieben werden.

Besonders gut geeignet sind als Trimerisierungskatalysatoren außerdem quaternäre Ammoniumhydroxide der allgemeinen Formel 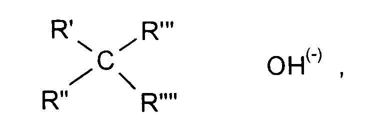
wie sie in den DE-OS'en 28 06 731 und 29 01 479 beschrieben sind. Bevorzugt werden dabei quaternäre Ammoniumhydroxide der angegebenen Struktur, wobei die Reste R' bis R'''' für gleiche oder verschiedene Alkyl-oder Aralkylgruppen mit 1 bis 20, vorzugsweise 1 bis 7, Kohlenstoffatomen stehen die gegebenenfalls mit Hydroxylgruppen substituiert sind und wobei zwei der genannten Reste R' bis R'''' auch zusammen mit dem Stickstoffatom und gegebenenfalls mit einem weiteren Stickstoff- oder Sauerstoffatom einen heterocyclischen Ring mit 3 bis 5 Kohlenstoffatomen bilden können oder wobei die Reste R' bis R''' jeweils für Ethylenreste stehen, die zusammen mit dem quaternären Stickstoffatom und einem weiteren tertiären Stickstoffatom ein bicyclisches Triethylendiamin-Gerüst bilden. Stellt einer oder mehrere Reste eine Hydroxyalkylgruppe dar, so weist er bevorzugt 2 bis 4 Kohlenstoffatome auf, wobei die Hydroxylgruppe vorzugsweise in 2-Stellung zum quaternären Stickstoffatom angeordnet ist, wobei in den genannten Fällen der hydroxylsubstituierte Rest oder die hydroxylsubstituierten Reste außer dem Hydroxyl-Substituenten auch beliebige andere Substituenten, insbesondere C₁- bis C₄-Alkoxy-Substituenten, aufweisen können.

Die Darstellung der zuletzt beschriebenen Katalysatoren erfolgt in an sich bekannter Weise durch Umsetzung von tertiärem Amin mit Alkylenoxid in wäßrigalkoholischem Medium (vgl. US-PS 39 95 997, Kol. 2, Zeilen 19-41). Als tertiäre Amine sind dafür beispielsweise genannt: Trimethylamin, Tributylamin, 2-Dimethylaminoethanol, Triethanolamin, Dodecyldimethylamin, N,N-Dimethylcyclohexylamin, N-Methylpyrrolidin, N-Methylmorpholin oder 1,4-Diazabicyclo-(2,2,2)-octan; als Alkylenoxide können beispielsweise Ethylenoxid, Propylenoxid, 1,2-Butylenoxid, Styroloxid oder Methoxy-, Ethoxy- oder Phenoxypropylenoxid verwendet werden. Ganz besonders bevorzugte Katalysatoren aus dieser Gruppe sind N,N,N-Trimethyl-N-(2-hydroxyethyl)-ammoniumhydroxid und N,N,N-Trimethyl-N-(2-hydroxypropyl)-ammoniumhydroxid. Besonders gut geeignet ist auch ein quaternäres Ammoniumhydroxid der angegebenen Formel mit

R' = R'' = R''' = CH₃ und R'''' = -CH₂-C₆H₅ .

Die Trimerisierungskatalysatoren werden im allgemeinen in Mengen von 0,005 bis 5 Gew.-%, vorzugsweise 0,01 bis 2 Gew.-%, bezogen auf bei der Trimerisierungsreaktion eingesetztes Ausgangsgemish eingesetzt. Wird z.B. ein bevorzugter Katalysator wie N,N,N-Trimethyl-N-(2-hydroxypropyl)-ammoniumhydroxid verwendet, so sind Mengen von 0,05 bis 1 Gew.-%, bevorzugt von 0,07 bis 0,7 Gew.-%, bezogen auf Ausgangsgemish im allgemeinen ausreichend. Die Katalysatoren können in reiner Form oder als Lösung eingesetzt werden.

Geeignete Lösemittel für die jeweils verwendeten Katalysatoren sind beispielsweise in den obengenannten Veröffentlichungen zitiert.

Bei der Mitverwendung von Carbamidsäurederivate-bildenden Hydroxyverbindungen als Cokatalysatoren ist es von Vorteil diese gleichzeitig als Katalysatorlösungsmittel zu verwenden. Dafür eignen sich z.B. Methanol, Ethanol, Isopropanol, Butanol, 2-Ethylhexanol oder Glykole wie 1,2-Ethandiol, 1,2-Propandiol, 1,3-und 1,4-Butandiol, 1,6- und 2,5-Hexandiol und 2-Ethyl-1,3-hexandiol.

Die zur Trimerisierung erforderliche Reaktionstemperatur liegt bei 20 bis 200°C, bevorzugt bei 40 bis 160°C. Die Trimerisierungsreaktion wird bevorzugt in Abwesenheit von Lösungsmitteln durchgeführt.

Gegebenenfalls kann die Trimerisierungsreaktion beim gewünschten Trimerisierungsgrad durch Zugabe eines Katalysatorengiftes und/oder thermische Desaktivierung des Katalysators abgebrochen werden. Geeignete Katalysatorengifte für die jeweiligen Trimerisierungskatalysatoren sind in den obengenannten Veröffentlichungen zitiert. In einer bevorzugten Ausführungsform wird beim erfindungsgemäßen Verfahren jedoch auf die Desaktivierung des Trimerisierungskatalysators verzichtet. Aufgrund der vergleichsweise niedrigen Reaktivität der tertiär gebundenen Isocyanatgruppen der Ausgangskomponenten A) kommt die Reaktion nach Trimerisierung sämtlicher im Reaktionsgemisch vorhandenen primären und gegebenenfalls sekundären Isocyanatgruppen im allgemeinen von selbst zum Stillstand.

Bei der Herstellung der erfindungsgemäßen Polyisocyanatgemische werden im übrigen Art und Mengenverhältnisse der Ausgangsmaterialien, sowie gegebenenfalls der Trimerisierungsgrad so gewählt, daß ein modifiziertes Polyisocyanat bzw. ein modifiziertes Polyiscyanatgemisch erhalten wird, welches den obengenannten Angaben a) bis f) entspricht, wobei der Gehalt der modifizierten Polyisocyanatgemische an tertiär gebundenen Isocyanatgruppen vorzugsweise bei 7 bis 20 Gew.-%, der Gehalt an primär und/oder sekundär gebundenen Isocyanatgruppen vorzugsweise unterhalb 0,5 Gew.-%, die mittlere NCO-Funktionalität vorzugsweise bei 2,1 bis 5,0, insbesondere bei 2,2 bis 4,8, der Gehalt an Urethangruppen vorzugsweise bei 3 bis 25 Gew.-%, der Gehalt an Isocyanuratgruppen vorzugsweise bei 0 bis 25 Gew.-%, der Gehalt an monomeren Ausgangsdiisocyanaten unter 1, vorzugsweise unter 0,5 Gew.-%, der Gehalt an Estergruppen vorzugsweise bei 0 bis 20 Gew.-% und der Gehalt an Carbonatgruppen vorzugsweise bei 0 bis 25 Gew.-% liegt, wobei der Gesamtgehalt an Estergruppen und Carbonatgruppen vorzugsweise mindestens 3 Gew.-% beträgt und wobei die modifizierten Polyisocyanatgemische unterhalb 40°C fest und oberhalb 125°C flüssig sind, insbesondere einen nach der Differential-Thermoanalyse (DTA) bestimmten Schmelzpunkt bzw. Schmelzbereich aufweisen, der innerhalb des Temperaturbereichs von 40 bis 110°C, besonders bevorzugt innerhalb des Temperaturbereichs von 50 bis 100°C liegt. Die erfindungsgemäßen Polyisocyanatgemische genügen im allgemeinen diesen Kriterien, wenn bei ihrer Herstellung bezüglich Auswahl der Ausgangsmaterialien, Wahl der Mengenverhältnisse der Ausgangsmaterialien und Durchführung der Modifizierungsreaktion bzw. der Modifizierungsreaktionen die oben gemachten Angaben beachtet werden wobei der Schmelzpunkt bzw. der Schmelzbereich beispielsweise durch Verwendung verzweigter Polyhydroxylverbindungen C) und gegebenenfalls D) erhöht, dementsprechend durch Mitverwendung von linearen Polyhydroxylverbindungen erniedrigt oder die NCO-Funktionalität durch Mitverwendung von höherfunktionellen Polyhydroxylverbindungen erhöht werden können.

Durch Mitverwendung unterschiedlicher Mengen an Diisocyanaten B) mit primären und/oder sekundären Diisocyanatgruppen, insbesondere an 1,6-Diisocyanatohexan, bei der Trimerisierungsreaktion kann ebenfalls die Funktionalität in gewünschter Weise eingestellt werden. Ferner ist der Isocyanuratgehalt der modifizierten Polyisocyanatgemische von unmittelbarem Einfluß auf deren Schmelzbereich, so daß durch den Trimerisierungsgrad, d.h. durch die Menge der vor der Trimerisierungsreaktion vorliegenden, primär gebundenen Isocyanatgruppen nicht nur die Funktionalität, sondern auch der optimale Schmelzbereich eingestellt werden kann.

Die erfindungsgemäßen Polyisocyanatgemische stellen wertvolle Ausgangsmaterialien zur Herstellung von Polyurethankunststoffen nach dem Isocyanat-Polyadditionsverfaliren dar. Sie finden insbesondere Verwendung als Vernetzerkomponente in hitzehärtbaren blockierungsmittelfreien Zweikomponenten-PUR-Pulverlacken.

Geeignete Reaktionspartner für die erfindungsgemäßen Polyisocyanatgemische sind dabei grundsätzlich sämtliche aus der Pulverlack-Technologie bekannten Bindemittel mit gegenüber Isocyanaten reaktionsfähigen Gruppen, wie z.B. Hydroxyl-, Carboxyl-, Amino-, Thiol-, Urethan- oder Harnstoffgruppen. Bevorzugt kommen jedoch hydroxyfunktionelle Pulverlackbindemittel zum Einsatz, die unterhalb 40°C fest und oberhalb 130°C flüssig sind. Die Erweichungstemperaturen dieser hydroxyfunktionellen Harze - bestimmt nach der Differential-Thermoanalyse (DTA) -liegen vorzugsweise innerhalb des Temperaturbereiches von 30 bis 120°C, besonders bevorzugt innerhalb des Temperaturbereiches von 35 bis 110°C.

Ihre Hydroxylzahlen liegen im allgemeinen zwischen 25 und 200, vorzugsweise zwischen 30 und 130 und ihr mittleres (aus der Funktionalität und dem Hydroxylgehalt erreichenbares) Molekulargewicht im allgemeinen zwischen 400 und 10.000, vorzugsweise zwischen 1.000 und 5.000.

Derartige Pulverlackbindemittel sind beispielsweise hydroxylgruppenhaltige Polyester, Polyacrylate oder Polyurethane, wie sie in den obengenannten Veröffentlichungen des Standes der Technik, z.B. EP-A-0 045 998 oder EP-A-0 254 152 beschrieben sind, aber auch beliebige Mischungen solcher Harze.

Zur Herstellung eines gebrauchsfertigen Pulverlacks werden die erfindungsgemäßen Polyisocyanatgemische mit geeigneten hydroxyfunktionellen Pulverlackbindemitteln gemischt, gegebenenfalls mit weiteren Hilfs- und Zusatzmitteln, wie z.B. Katalysatoren, Pigmenten, Füllstoffen oder Verlaufsmitteln versetzt, und beispielsweise auf Extrudern oder Knetern oberhalb des Schmelzbereichs der Einzelkomponenten, beispielsweise 70 bis 130°C, zu einem homogenen Material vereinigt.

Der nach Abkühlen der Schmelze resultiernde Feststoff wird anschließend gemahlen und durch Sieben von den Kornanteilen oberhalb der gewünschten Korngröße, beispielsweise oberhalb 0,1 mm, befreit.

Die erfindungsgemäßen Polyisocyanatgemische und die hydroxyfunktionellen Bindemittel kommen hierbei in solchen Mengenverhältnissen zum Einsatz, daß auf jede Hydroxylgruppe 0,6 bis 1,2, vorzugsweise 0,8 bis 1,0, Isocyanatgruppen entfallen.

Bei den gegebenenfalls zur Beschleunigung der Aushärtung mitzuverwendenden Katalysatoren handelt es sich um die üblichen, aus der Polyurethanchemie bekannten Verbindungen, wie sie schon oben beim erfindungsgemäßen Verfahren zur Katalyse der Urethanisierungsreaktion beschrieben wurden. Diese Katalysatoren können gegebenenfalls in Mengen von 0,01 bis 5,0 Gew.-%, vorzugsweise 0,05 bis 1,0 Gew.-%, bezogen auf die Gesamtmenge an organischem Bindemittel, d.h. erfindungsgemäßen Polyisocyanatgemischen in Kombination mit den hydroxyfunktionellen Pulverlackbindemitteln aber exclusive den gegebenenfalls verwendeten weiteren Hilfs- und Zusatzmitteln, zugesetzt werden.

Der so hergestellte sprühfertige Pulverlack kann nach üblichen Pulverauftragsverfahren, wie z.B. elektrostatisches Pulverversprühen oder Wirbelsintern, auf die zu überziehenden Substrate aufgebracht werden. Die Härtung der Überzüge erfolgt durch Erhitzen auf Temperaturen von 100 bis 200°C, vorzugsweise 120 bis 160°C beispielsweise während eines Zeitraums von ca. 10 bis 30 Minuten. Man erhält bereits bei niedrigen Einbrenntemperaturen glänzende Beschichtungen hoher Härte und Elastizität, die sich durch hervorragende Lösemittel-und Chemikalienbeständigkeit sowie eine sehr gute Wärmefarbstabilität auszeichnen.

Mit Hilfe der erfindungsgemäßen Polyisocyanatgemische hergestellte Pulverlacke weisen eine hohe Licht- und Wetterbeständigkeit auf, und sind somit für Außenanwendungen besonders geeignet.

Erfindungsgemäß können beliebige hitzeresistente Substrate, wie beispielsweise solche aus Glas oder Metallen, beschichtet werden.

Neben der beschriebenen Verwendung als Pulverlackhärter lassen sich die erfindungsgemäßen Polyisocyanatgemische, gelöst in üblichen Lacklösemitteln, wie z.B. Estern, Ketonen oder Kohlenwasserstoffen, und gegebenenfalls auch in mit Blockierungsmitteln blockierter Form, selbstverständlich auch als Vernetzerkomponente für lösemittelhaltige ein- bzw. zweikomponentige Polyurethanlacke einsetzen.

Die nachfolgenden Beispiele dienen zur weiteren Erläuterung der Erfindung. Alle Prozentangaben, mit Ausnahme der Glanzwerte, beziehen sich auf Gewichtsprozente.

### Beispiele

### Herstellung der Ausgangsverbindungen C

### Estergruppen-haltiges Triol C1

2546 g 1,1,1-Trimethylolpropan (TMP) und 1431 g ε-Caprolacton werden bei Raumtemperatur unter trockenem Stickstoff vermischt, mit 0,04 g ortho-Phosphorsäure versetzt und anschließend für 5 h auf 160°C erhitzt. Nach Abkühlen auf Raumtemperatur erhält man ein farbloses, flüssiges Produkt mit folgenden Kenndaten:

| | |
|---|---|
| η (23°C): | 3800 mPa.s |
| OH-Zahl: | 790 mg KOH/g |
| freies Caprolacton: | 0,2 % |
| mittleres Molekulargewicht (aus OH-Zahl ber.): | 213 |
| Estergruppengehalt (ber.): | 13,9 % |

### Estergruppen-haltiges Triol C2

590 g 1,1,1-Trimethylolpropan (TMP) und 1505 g ε-Caprolacton werden bei Raumtemperatur unter trockenem Stickstoff vermischt mit 0,02 g ortho-Phosphorsäure versetzt und anschließend für 5 h auf 160°C erhitzt. Nach Abkühlen auf Raumtemperatur erhält man ein farbloses, flüssiges Produkt mit folgenden Kenndaten:

| | |
|---|---|
| η (23°C): | 1400 mPa.s |
| OH-Zahl: | 341 mg KOH/g |
| freies Caprolacton: | 0,4 % |
| mittleres Molekulargewicht (aus OH-Zahl ber.): | 493 |
| Estergruppengehalt (ber.): | 27,7 % |

### Estergruppen-haltiges Diol C3

254 g 1,6-Hexandiol und 1146 g ε-Caprolacton werden bei Raumtemperatur unter trockenem Stickstoff vermischt, mit 0,07 g Zin(II)-octoat versetzt und anschließend für 4 h auf 160°C erhitzt. Nach Abkühlen auf Raumtemperatur erhält man ein farbloses, flüssiges Produkt mit folgenden Kenndaten:

| | |
|---|---|
| η (23°C): | 330 mPa.s |
| OH-Zahl: | 172 mg KOH/g |
| freies Caprolacton: | 0,5 % |
| mittleres Molekulargewicht (aus OH-Zahl ber.): | 651 |
| Estergruppengehalt (ber.): | 31,6 % |

### Estergruppen-haltiges Triol C4

600 g 1,1,1-Trimethylolethan und 1140 g ε-Caprolacton werden bei Raumtemperatur unter trockenem Stickstoff vermischt, mit 0,09 g Zinn(II)-octoat versetzt und anschließend für 5 h auf 160°C erhitzt. Nach Abkühlen auf Raumtemperatur erhält man ein farbloses, flüssiges Produkt mit folgenden Kenndaten:

| | |
|---|---|
| η (23°C): | 1920 mPa.s |
| OH-Zahl: | 468 mg KOH/g |
| freies Caprolacton: | 0,8 % |
| mittleres Molekulargewicht (aus OH-Zahl ber.): | 359 |
| Estergruppengehalt (ber.): | 25,3 % |

### Beispiel 1

1940 g (10,0 mol) 1-Isocyanato-1-methyl-4(3)-isocyanatomethylcyclohexan (IMCI) werden unter trockenem Stickstoff auf 80°C erwärmt. Anschließend gibt man innerhalb von 2 bis 3 Stunden 851 g (4,0 mol) des Estergruppen-haltigen Triols C1 unter Rühren so zu, daß die Reaktionstemperatur 110°C nicht übersteigt (NCO/OH-Äquivalentverhältnis = 1,7:1). Nach beendeter Zugabe wird weitere 2 bis 3 Stunden bei 100 bis 110°C gerührt, bis der theoretisch berechnete NCO-Gehalt erreicht ist. Man erhält ein erfindungsgemäßes Polyisocyanatgemisch in Form eines farblosen Festharzes mit einem NCO-Gehalt von 12,0 %, einem Restgehalt an monomerem IMCI von 0,3 % und einem Schmelzpunkt von 65 bis 68°C. Der Gehalt an Urethangruppen beträgt 25,3 % und an Estergruppen 4,2 %.

### Beispiel 2

776,0 g (4,0 mol) IMCI werden bei 60°C innerhalb von 30 min mit 190,4 g (0,39 mol) des Estergruppen-haltigen Triols C2 unter Rühren versetzt, wobei die Innentemperatur durch die exotherme Reaktion bis 80°C ansteigt. Man rührt 1 h bei 80°C nach, bis ein NCO-Gehalt von 29,7 % erreicht ist. Nach Abkühlen auf 40°C werden 67,2 g (0,4 mol) Hexamethylendiisocyanat (HDI) zugegeben, die klare Lösung wird im Vakuum entgast und mit N₂ belüftet. Zur Trimerisierung werden innerhalb 5 min bei 40°C 3 g einer Katalysatorlösung (5 %ige Lösung von Trimethyl-benzyl-ammoniumhydroxid in 1,3-Butandiol) zugetropft. Man heizt bis 60°C und hält die exotherme Umsetzung zunächst durch leichtes Kühlen, später durch Heizen bei dieser Temperatur, bis nach ca. 3 h ein NCO-Gehalt von 21 % unterschritten ist; nach ca. 2,5 h werden weitere 1,5 g der Kataysatorlösung zugegeben. Jetzt wird während weiterer 2 h die Temperatur allmählich auf 80 bis 90°C, gegen Reaktionsende bis 110°C erhöht, wobei nach 1 h bei 90°C nochmals 1,5 g Katalysatorlösung nachgegeben werden. Nach beendeter Umsetzung läßt man die Schmelze erkalten und erhält ein erfindungsgemäßes farbloses Festharz mit einem NCO-Gehalt von 14,5 %, einem Restgehalt an monomerem IMCI von 0,4 %, an monomerem HDI von ≦0,03 % und einem Schmelzpunkt von 58 bis 63°C. Der Gehalt an Urethangruppen beträgt 7,3 %, an Estergruppen 5,1 % und an Isocyanuratgruppen 15,8 %.

### Beispiel 3

1940 g (10 mol) IMCI werden analog Beispiel 1 mit einer Mischung aus 252 g (0,39 mol) Estergruppen-haltigem Diol C3 und 502 g (3,7 mol) TMP umgesetzt. Man erhält ein erfindungsgemäßes Polyisocyanatgemisch in Form eines farblosen Festharzes mit einem NCO-Gehalt von 12,4 %, einem Restgehalt an monomerem IMCI von 0,3 % und einem Schmelzpunkt von 86 bis 90°C. Der Urethangruppengehalt beträgt 26,3 %, der Estergruppengehalt 2,9 %.

### Beispiel 4

Eine Mischung aus 970 g (5 mol) IMCI und 168 g (1 mol) HDI wird analog Beispiel 1 mit einer Mischung aus 557 g (1,6 mol) Estergruppen-haltigem Triol C4 und 257 g (1,8 mol) 1,4-Cyclohexandimethanol umgesetzt. Man erhält ein erfindungsgemäßes Polyisocyanatgemisch in Form eines praktisch farblosen Festharzes mit einem NCO-Gehalt von 8,0 %, einem Restgehalt an monomerem IMCI von 0,3 % bzw. an monomerem HDI von 0,1 % und einem Schmelzbereich von 82 bis 87°C. Der Gehalt an Estergruppen beträgt 7,2 % und an Urethangruppen 24,9 %.

### Beispiel 5

970 g (5,0 Mol) IMCl werden analog Beispiel 1 mit einer Mischung aus 300 g (0,15 Mol) eines durch Reaktion von 1,6-Hexandiol mit Diphenylcarbonat hergestellten Polycarbonatdiols vom Molekulargewicht 2000 und 255 g (1,9 Mol) TMP umgesetzt. Man erhält ein erfindungsgemäßes Polyisocyanatgemisch in Form eines farblosen Festharzes mit einem NCO-Gehalt von 11,0 %, einem Restgehalt an monomeren IMCl von 0,4 % und einem Schmelzpunkt von 64 bis 66°C. Der Urethangruppengehalt beträgt 23,2 %, der Carbonatgruppengehalt 7,7 %.

### Beispiel 6 (Verwendung)

75,6 Gew.-Teile eines hydroxylgruppenhaltigen Polyesters, der aus 66,6 Gew.-Teilen Terephthalsäure, 38,2 Gew.-Teilen Neopentylglykol, 5,3 Gew.-Teilen 1,6-Hexandiol und 4,5 Gew.-Teilen 1,1,1-Trimethylolpropan hergestellt wurde und eine OH-Zahl von 50 und einen Schmelzbereich (bestimmt nach der Differential-Thermoanalyse) von 55 bis 60°C aufweist, werden mit 23,4 Gew.-Teilen des gemäß Beispiel 1 erhaltenen erfindungsgemäßen Polyisocyanatgemisches, entsprechend einem NCO/OH-Äquivalentverhältnis von 1:1 und 1,0 Gew.-Teilen eines handelsüblichen Verlaufsmittels (®Perenol F 30 P; Fa. Henkel, Düsseldorf) gründlich gemischt und anschließend mit Hilfe eines Buss Cokneters vom Typ PLK 46 bei 150 U/min und einer Gehäusetemperatur von 40°C im Einzugsbereich sowie an der Welle bzw. von 80°C im Verfahrensteil homogenisiert, wobei Massetemperaturen von 93 bis 98°C erreicht werden. Die erstarrte Schmelze wird mit Hilfe einer Sichtermühle ACM 2 (Fa. Hosokawa Mikropul) mit einem 90 m Sieb gemahlen und gesiebt. Das so erhaltene Pulver wird mit einer ESB Becherpistole bei einer Hochspannung von 70 kv auf ein entfettetes Stahlblech gespritzt und jeweils 30 min bei 140°C und 160°C zu einer glattverlaufenden transparenten Beschichtung ausgehärtet.

Bei einer Schichtdicke von ca. 55 m werden folgende lacktechnische Eigenschaften gefunden:

| | 140°C | 160°C |
|---|---|---|
| Glanz: (Gardner, 60°C Reflexionswinkel) | 100 % | 103 % |
| Erichsentiefung: (DIN 53 156) | >9,0 mm | >9,0 mm |
| Gelierzeit: (DIN 55 990 Teil 8, Punkt 5.1) | 212 sec/180°C | |

Der Versuch zeigt, daß bereits nach 30 min/140°C ein vollvernetzter, elastischer Lackfilm erhalten wird.

### Beispiel 7 (Verwendung)

Aus 78,8 Gew.-Teilen des in Beispiel 6 beschriebenen hydroxylgruppenhaltigen Polyesters, 20,2 Gew.-Teilen des gemäß Beispiel 2 erhaltenen erfindungsgemäßen Polyisocyanatgemisches (NCO/OH-Äquivalentverhältnis = 1:1) sowie 1,0 Gew.-Teilen eines handelsüblichen Verlaufsmittels (®Perenol F 30 P; Fa. Henkel, Düsseldorf) wird nach der in Beispiel 6 beschriebenen Methode ein Pulverklarlack formuliert, auf ein entfettetes Stahlblech gespritzt und 30 min bei 140°C zu einer glattverlaufenden transparenten Beschichtung eingebrannt.

Bei einer Schichtdicke von ca. 60 µm werden folgende lacktechnischen Eigenschaften gefunden:

| | |
|---|---|
| Glanz: | 99 % |
| Erichsentiefung: | >9,0 mm |
| Gelierzeit: | 152 sec/180°C |

### Beispiel 8 (Verwendung)

Aus 68,9 Gew.-Teilen eines hydroxylgruppenhaltigen Polyacrylates, bestehend aus 37,0 Gew.-Teilen Methylmethacrylat, 24,0 Gew.-Teilen n-Butylmethacrylat, 18,9 Gew.-Teilen Styrol, 19,1 Gew.-Teilen 2-Hydroxyethylmethacrylat und 1,0 Gew.-Teilen Acrylsäure mit einer OH-Zahl von 70 und einem Schmelzbereich (bestimmt nach der Differential-Thermoanalyse) von 62 bis 66°C, 30,1 Gew.-Teilen des gemäß Beispiel 1 erhaltenen erfindungsgemäßen Polyisocyanatgemisches (NCO/OH-Äquivalentverhaltnis = 1:1) sowie 1,0 Gew.-Teilen eines handelsüblichen Verlaufsmittels (®Perenol F 30 P; Fa. Henkel, Düsseldorf) wird nach der in Beispiel 6 beschriebenen Methode ein Pulverklarlack formuliert, auf ein entfettetes Stahlblech gespritzt und 30 min bei 140°C zu einer glattverlaufenden transparenten Beschichtung eingebrannt.

Bei einer Schichtdicke von ca. 60 µm werden folgende lacktechnische Eigenschaften gefunden:

| | |
|---|---|
| Glanz: | 90% |
| Acetonbeständigkeit: (50 Doppelhübe mit getränktem Wattebausch) | 0 |
| Bewertung: | 0 = Film intakt |
| | 1 = Filmoberfläche angeweicht |
| | 2 = Film bis zum Untergrund angequollen) |
| Gelierzeit: | 65 sec/180°C |

Der Versuch zeigt, daß bereits nach 30 min/140°C ein lösemittelfester Lackfilm erhalten wird.

### Beispiel 9 (Verwendung)

Aus 70,1 Gew.-Teilen des in Beispiel 8 beschriebenen hydroxylgruppenhaltigen Polyacrylates, 28,9 Gew.-Teilen des gemäß Beispiel 2 erhaltenen erfindungsgemäßen Polyisocyanatgemisches (NCO/OH-Äquivalentverhältnis = 1:1) sowie 1,0 Gew.-Teilen eines handelsüblichen Verlaufsmittels (®Perenol F 30 P; Fa. Henkel, Düsseldorf) wird nach der in Beispiel 6 beschriebenen Methode ein Pulverklarlack formuliert, auf ein entfettetes Stahlblech gespritzt und 30 min bei 140°C zu einer glattverlaufenen transparenten Beschichtung eingebrannt.

Bei einer Schichtdicke von ca. 55 µm werden folgende lacktechnischen Eigenschaften gefunden:

| | |
|---|---|
| Glanz: | 93 % |
| Acetonbeständigkeit: | 0 |
| Gelierzeit: | 44 sec/180°C |

### Beispiel 10 (Verwendung)

Aus 67,0 Gew.-Teilen des in Beispiel 8 beschriebenen hydroxylgruppenhaltigen Polyacrylates, 32,0 Gew.-Teilen des gemäß Beispiel 2 erhaltenen erfindungsgemäßen Polyisocyanatgemisches (NCO/OH-Äquivalentverhältnis = 1:1) sowie 1,0 Gew.-Teilen eines handelsüblichen Verlaufsmittels (®Perenol F 30 P; Fa. Henkel, Düsseldorf) wird nach der in Beispiel 6 beschriebenen Methode ein Pulverklarlack formuliert, auf ein entfettetes Stahlblech gespritzt und 30 min bei 140°C zu einer glattverlaufenen transparenten Beschichtung eingebrannt.

Bei einer Schichtdicke von ca. 55 µm werden folgende lacktechnischen Eigenschaften gefunden:

| | |
|---|---|
| Glanz: | 94 % |
| Acetonbeständigkeit: | 0 |
| Gelierzeit: | 61 sec/180°C |

## Patentansprüche

1. Polyisocyanatgemische, die unterhalb von 40°C in fester und oberhalb von 125°C in flüssiger Form vorliegen und
a) eine mittlere Isocyanatfunktionalität von mindestens 2,1,
b) einen Gehalt an freien, an tertiäre (cyclo)aliphatische Kohlenstoffatome gebundenen Isocyanatgruppen (berechnet als NCO; Molekulargewicht = 42) von 5 bis 22 Gew.-%,
c) einen Gehalt an Urethangruppen (berechnet als -NH-CO-O-; Molekulargewicht = 59) von 2 bis 30 Gew.-% und
d) einen Gehalt an Isocyanuratgruppen (berechnet als C₃N₃O₃; Molekulargewicht = 126) von 0 bis 30 Gew.-%
aufweisen, dadurch gekennzeichnet, daß die Polyisocyanatgemische zusätzlich
e) einen Gehalt an Carbonsäureestergruppen (berechnet als -CO-O-, Molekulargewicht = 44) von 0 bis 25 Gew.-% und/oder
f) einen Gehalt an Carbonatgruppen (berechnet als -O-CO-O-; Molekulargewicht = 60) von 0 bis 34 Gew.-% aufweisen,
mit der Maßgabe, daß der Gesamtgehalt an Carbonsäureester- und Carbonatgruppen mindestens 2 Gew.-% beträgt.

2. Verfahren zur Herstellung von Polyisocyanatgemischen gemäß Anspruch 1, dadurch gekennzeichnet, daß man
A) Diisocyanate, die mindestens eine an ein tertiäres (cyclo)aliphatisches Kohlenstoffatom gebundene Isocyanatgruppe aufweisen, gegebenenfalls unter Mitverwendung von
B) weiteren Diisocyanaten mit ausschließlich an primären und/oder sekundären (cyclo)aliphatischen Kohlenstoffatomen gebundenen Isocyanatgruppen mit
C) Estergruppen und/oder Carbonatgruppen aufweisenden Polyhydroxylverbindungen eines mittleren Molekulargewichtes von 134 bis 1200, gegebenenfalls unter gleichzeitiger Mitverwendung von
D) Estergruppen- und Carbonatgruppen-freien Polyhydroxylverbindungen des Molekulargewichtsbereiches 62 bis 400,
unter Einhaltung eines NCO/OH-Äquivalentverhältnisses von 1,2:1 bis 40:1 bis zum Erreichen des theoretisch berechneten NCO-Gehaltes umsetzt, gegebenenfalls im Anschluß daran die Umsetzungsprodukte durch katalytische Trimerisierung eines Teils der verbliebenen Isocyanatgruppen weiter modifiziert und/oder gegebenenfalls überschüssiges, nicht umgesetztes Diisocyanat durch Dünnschichtdestillation abtrennt, wobei im übrigen Art und Mengenverhältnisse der genannten Ausgangsstoffe so gewählt werden, daß die resultierenden Verfahrensprodukte den in Anspruch 1 unter a) bis f) genannten Bedingungen entsprechen.

3. Verfahren gemäß Anspruch 2, dadurch gekennzeichnet, daß man als Diisocyanate A)
A1) aliphatische Diisocyanate mit einem NCO-Gehalt von 24 bis 50 Gew.-%, die neben einer sterisch ungehinderten, an ein primäres aliphatisches Kohlenstoffatom gebundenen Isocyanatgruppe eine sterisch gehinderte Isocyanatgruppe, die an ein tertiäres aliphatisches Kohlenstoffatom gebunden ist aufweisen,
A2) aliphatisch-cycloaliphatische Diisocyanate mit einem NCO-Gehalt von 20 bis 50 Gew.-%, die neben einer sterisch ungehinderten, an ein primäres aliphatisches Kohlenstoffatom gebundenen Isocyanatgruppe eine sterisch gehinderte Isocyanatgruppe, die an ein tertiäres Kohlenstoffatom, das Teil eines cycloaliphatischen Ringsystems ist, gebunden ist, aufweisen, oder
A3) zwei Isocyanatoalkyl-Substituenten aufweisende aromatische Verbindungen mit einem NCO-Gehalt von 20 bis 35 Gew.-%, bei denen beide Isocyanatgruppen an tertiäre aliphatische Kohlenstoffatome gebunden sind,
oder beliebige Gemische solcher Diisocyanate verwendet.

4. Verfahren gemäß Anspruch 2 und 3, dadurch gekennzeichnet, daß man als Diisocyanat A) 1-Isocyanato-1-methyl-4(3)-isocyanatomethylcyclohexan verwendet.

5. Verwendung der Polyisocyanatgemische gemäß Anspruch 1 als Ausgangskompnente bei der Herstellung von Polyurethankunststoffen.

6. Verwendung gemäß Anspruch 5 als Vernetzerkomponente in hitzehärtbaren Zweikomponenten-Polyurethan-Pulverlacken bei der Beschichtung beliebiger hitzeresistenter Substrate nach den Methoden der Pulverlacktechnologie.

## Claims

1. Polyisocyanate mixtures which are in solid form below 40°C and in liquid form above 125°C and which have
a) an average isocyanate functionality of at least 2.1,
b) a content of free isocyanate groups attached to tertiary (cyclo)aliphatic carbon atoms (calculated as NCO; molecular weight = 42) of 5 to 22 wt.%,
c) a urethane group content (calculated as -NH-CO-O-; molecular weight = 59) of 2 to 30 wt.% and
d) an isocyanurate group content (calculated as C₃N₃O₃; molecular weight = 126) of 0 to 30 wt.%,
characterised in that the polyisocyanate mixtures additionally have
e) a carboxylic acid ester group content (calculated as -CO-O-; molecular weight = 44) of 0 to 25 wt.% and/or
f) a carbonate group content (calculated as -O-CO-O-; molecular weight = 60) of 0 to 34 wt.%,
providing that the total content of carboxylic acid ester and carbonate groups is at least 2 wt.%.

2. Process for the production of polyisocyanate mixtures according to claim 1, characterised in that
A) diisocyanates having at least one isocyanate group attached to a tertiary (cyclo)aliphatic carbon atom, optionally also using
B) further diisocyanates with isocyanate groups exclusively attached to primary and/or secondary (cyclo)aliphatic carbon atoms are reacted with
C) polyhydroxyl compounds with ester groups and/or carbonate groups and of an average molecular weight of 134 to 1200, optionally simultaneously also using
D) polyhydroxyl compounds containing neither ester nor carbonate groups and of the molecular weight range from 62 to 400,
while maintaining an NCO/OH equivalent ratio of 1.2:1 to 40:1 until the theoretically calculated NCO content is reached, optionally thereafter further modifying the reaction products by catalytic trimerisation of a proportion of the remaining isocyanate groups and/or optionally separating excess, unreacted diisocyanate by film distillation, wherein the type and quantity ratios of the stated starting materials are moreover selected such that the products resulting from the process comply with the requirements stated in claim 1 under a) to f).

3. Process according to claim 2, characterised in that
A1) aliphatic diisocyanates with an NCO content of 24 to 50 wt.% which, apart from a sterically unhindered isocyanate group attached to a primary aliphatic carbon atom, have a sterically hindered isocyanate group which is attached to a tertiary aliphatic carbon atom,
A2) aliphatic-cycloaliphatic diisocyanates with an NCO content of 20 to 50 wt.% which, apart from a sterically unhindered isocyanate group attached to a primary aliphatic carbon atom, have a sterically hindered isocyanate group which is attached to a tertiary carbon atom which is part of a cycloaliphatic ring system, or
A3) aromatic compounds with two isocyanatoalkyl substituents and with an NCO content of 20 to 35 wt.% in which both isocyanate groups are attached to tertiary aliphatic carbon atoms,
or any desired mixtures of such diisocyanates are used as diisocyanates A).

4. Process according to claims 2 and 3, characterised in that 1-isocyanato-1-methyl-4(3)-isocyanatomethylcyclohexane is used as diisocyanate A).

5. Use of the polyisocyanate mixtures according to claim 1 as a starting component in the production of polyurethane plastics.

6. Use according to claim 5 as a crosslinking component in heat-curable two-component polyurethane powder coatings for coating any desired heat-resistant substrates using powder coating technology methods.

## Revendications

1. Mélanges de polyisocyanates qui sont présents sous forme solide au-dessous de 40°C et sous forme liquide au-delà de 125°C, et qui présentent
a) un nombre moyen de groupes isocyanates d'au moins de 2,1,
b) une teneur en des groupes isocyanates libres, fixés sur des atomes de carbone (cyclo)aliphatiques tertiaires (nombre calculés en NCO ; poids moléculaire = 42) de 5 à 22 % en poids,
c) une teneur en des groupes uréthanes (calculée en -NH-CO-O- ; poids moléculaire = 59) de 2 à 30 % en poids, et
d) une teneur en des groupes isocyanurates (calculée en C₃N₃O₃ ; poids moléculaire = 126) de 0 à 30 % en poids,
ces mélanges étant caractérisés en ce que les mélanges de polyisocyanates présentent en outre
e) une teneur en des groupes esters d'acides carboxyliques (calculée en -CO-O-, poids moléculaire = 44) de 0 à 25 % en poids et/ou
f) une teneur en des groupes carbonates (calculée en -O-CO-O- ; poids moléculaire 60) de 0 à 34 % en poids,
étant bien entendu que la teneur totale en des groupes esters d'acide carboxylique et en des groupes carbonates vaut au moins 2 % en poids.

2. Procédé pour préparer des mélanges de polyisocyanates selon la revendication 1, procédé caractérisé en ce que l'on fait réagir
A) des diisocyanates, présentant au moins un groupe isocyanate fixé sur un atome de carbone (cyclo)aliphatique tertiaire, éventuellement avec co-utilisation de
B) d'autres diisocyanates comportant des groupes isocyanates fixés exclusivement sur des atomes de carbone (cyclo)aliphatiques primaires et/ou secondaires avec
C) des composés polyhydroxylés présentant des groupes esters et/ou des groupes carbonates et ayant un poids moléculaire moyen de 134 à 1200, éventuellement avec co-utilisation simultanée de
D) des composés polyhydroxylés dépourvus de groupes esters et de groupes carbonates et dont le domaine de poids moléculaire se situe entre 62 et 400,
avec maintien d'un rapport entre les équivalents de NCO/OH de 1,2:1 à 40:1 jusqu'à de la teneur théoriquement calculée en NCO, puis éventuellement, on modifie encore ensuite les produits de réaction par une trimérisation catalytique d'une partie des groupes isocyanates présents et/ou on sépare par distillation en couche mince le diisocyanate en excès n'ayant pas réagi, et l'on choisit de façon usuelle la nature et les rapports entre les quantités des matières citées de départ de façon que les produits résultat du procédé répondent aux conditions indiquées en a) à f) à la revendication 1.

3. Procédé selon la revendication 2, caractérisé en ce qu'on utilise comme diisocyanates (A) :
A1) des diisocyanates aliphatiques ayant une teneur en NCO de 24 à 50% en poids, et qui, en plus d'un groupe isocyanate, fixé sur un atome de carbone aliphatique primaire et ne présentant pas d'empêchement stérique, comportent un groupe isocyanate à empêchement stérique, et qui est fixé sur un atome de carbone aliphatique tertiaire,
A2) des diisocyanates aliphatiques-cycloaliphatiques ayant une teneur en NCO de 20 à 50 % en poids, et qui, en plus d'un groupe isocyanate ne présentant pas d'empêchement stérique et qui est fixé sur un atome de carbone aliphatique primaire, présentent un groupe isocyanate présentant de l'empêchement stérique et qui est fixé sur un atome de carbone tertiaire et qui fait partie d'un système cycloaliphatique, ou
A3) des composés aromatiques présentant deux substituants isocyanatoalkyles et ayant une teneur en NCO de 20 à 35 % en poids, dans le cas desquels les deux groupes isocyanates sont fixés sur des atomes de carbone aliphatiques tertiaires, ou
des mélanges quelconques de tels diisocyanates.

4. Procédé selon les revendications 2 et 3, caractérisé en ce qu'on utilise comme diisocyanate (A) le 1-ixocyanato-1-méthyl-4(3)-isocyanatométhylcyclohexane.

5. Utilisation des mélanges de polyisocyanates selon la revendication 1 comme composant de départ pendant la préparation de matières plastiques du type polyuréthane.

6. Utilisation selon la revendication 5, a titre de composant à rôle de réticulation, dans des laques et vernis en poudre de type polyuréthane à deux composants, thermodurcissables, pour le revêtement, selon les méthodes de technologie des laques et vernis en poudre, de substrats quelconques pouvant bien résister à la chaleur.
